(19) Europäisches Patentamt
European Patent Office
Office européen des brevets

(11) Publication number: 0 000 150
B1

(12) # EUROPEAN PATENT SPECIFICATION

(45) Date of publication of patent specification: 20.05.81

(21) Application number: 78100165.6

(22) Date of filing: 15.06.78

(51) Int. Cl.³: C 07 D 417/04, C 07 D 413/04, A 61 K 31/44

(54) **Dihydropyridine derivatives, process for their production and pharmaceutical compositions containing them.**

(30) Priority: 20.06.77 CH 7520/77
16.03.78 CH 2865/78

(43) Date of publication of application:
10.01.79 Bulletin 79/1

(45) Publication of the grant of the European patent:
20.05.81 Bulletin 81/20

(84) Designated Contracting States:
BE CH DE FR GB LU NL SE

(58) References cited:
None

(73) Proprietor: SANDOZ AG
Lichtstrasse 35
CH-4002 Basel (CH)

(72) Inventor: Neumann, Peter, Dr.
Kornhausstrasse 4
CH-3013 Berne (CH)

EP 0 000 150 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Dihydropyridine derivatives, process for their production and phamaceutical compositions containing them

The present invention relates to dihydropyridine derivatives.

The present invention provides compounds of formula I,

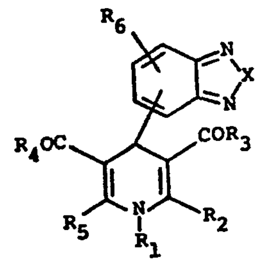

I

wherein

$R_1$ is hydrogen, alkyl or 1 to 6 carbon atoms, alkenyl or alkynyl or 3 to 6 carbon atoms, cycloalkyl of 3 to 7 carbon atoms, cycloalkylalkyl of 4 to 8 carbon atoms, phenylalkyl of 7 to 9 carbon atoms or phenylalkenyl of 9 to 12 carbon atoms, the phenyl ring being unsubstituted or mono, di- or trisubstituted independently by haloen, hydroxy or alkyl or alkoxy of 1 to 4 carbon atoms,

$R_2$ and $R_5$, independently, are hydrogen or alkyl of 1 to 6 carbon atoms,

$R_3$ and $R_4$ independently, are alkyl of 1 to 6 carbon atoms, alkenyl or alkynyl of 3 to 6 carbon atoms, cycloalkyl of 3 to 7 carbon atoms, cycloalkylalkyl of 4 to 8 carbon atoms, alkoxy of 1 to 6 carbon atoms, hydroxyalkoxy of 2 to 6 carbon atoms, alkoxyalkoxy of 3 to 6 carbon atoms, hydroxyalkoxyalkoxy of 4 to 8 carbon atoms, alkenyloxy or alkynyloxy of 3 to 6 carbon atoms, cycloalkyloxy of 3 to 7 carbon atoms or cycloalkylalkoxy of 4 to 8 carbon atoms,

$R^6$ is hydrogen, halogen, alkyl or alkoxy or alkylthio or alkylsulfonyl, each of 1 to 4 carbon atoms, trifluoromethyl, nitro or hydroxy, and

X is oxygen or sulphur.

In any of the above radicals alkyl of 1 to 6 carbon atoms is preferably of 1 to 4 carbon atoms, especially of 1 to 2 carbon atoms. Any alkyl, alkoxy, alkylthio or or alkylsulfonyl radical of 1 to 4 carbon atoms is preferably of 1 or 2 carbon atoms. The alkyl moiety of cycloalkylalkyl or cycloalkyalkoxy is conveniently methyl. Halogen means fluorine, chlorine or bromine and is especially chlorine. Cycloalkyl or the cycloalkyl moiety of cycloalkylalkyl or cycloalkylalkoxy is conveniently cyclopropyl or cyclopentyl or cyclohexyl. The multiple bond of alkenyl, alkynyl, alkenyloxy, alkynyloxy or phenylalkenyl is preferably not in the $\alpha$, $\beta$ position. Alkenyl, alkenyloxy, alkynyl or alkynyloxy preferably has 3 to 5 carbon atoms. Alkenyl or the alkenyl moiety of alkenyloxy is conveniently allyl or 2-methylallyl. Alkynyl or the alkynyl moiety of alkynyloxy is conveniently propynyl. Phenylalkenyl preferably has the trans-configuration and is for example cinnamyl. When $R_1$ is optionally substituted phenylalkyl, the phenyl group is preferably unsubstituted. When the phenyl group is di- or tri-substituted, preferably the substituents are the same. When $R_3$ and/or $R_4$ is alkoxy, this is preferably ethoxy or methoxy. When $R_3$ and/or $R_4$ is alkoxyalkoxy or hydroxyalkoxyalkoxy, preferably the carbon chain between the two ether oxygen atoms is of 2 carbon atoms. The hydroxy group of hydroxyalkoxy or of hydroxyalkoxyalkoxy is preferably not attached to the carbon atom attached to an ether oxygen atom. $R_1$ is preferably hydrogen. $R_2$ is conveniently identical to $R_5$. $R_2$ and/or $R_5$ is preferably methyl. $R_3$ and/or $R_4$ is preferably alkoxy or alkoxyalkoxy, especially n-butyloxyethoxy. $R_6$ is conveniently halogen, alkyl or alkoxy, or especially hydrogen. $R_6$ is conveniently adjacent to the dihydropyridine moiety which in turn is conveniently in the 4-position.

The present invention also provides a process for the production of a compound of formula I as defined above, comprising replacing the moiety —HC=Y in a compound of formula II,

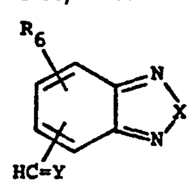

II

wherein $R_6$ and X are as defined above, and —HC=Y is

i) formyl,

ii) a radical of formula

—HC=C—C(=Z)$R_2$
|
$COR_3$

or
iii) a radical of formula

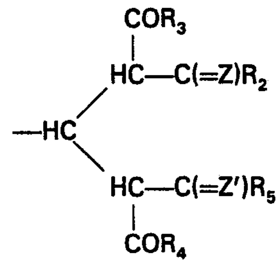

wherein z and z′ are independently oxygen or $NR_1$, and $R_1$ to $R_5$ are as defined above, by a moiety of formula III,

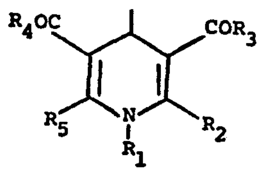

III

wherein $R_1$ to $R_5$ are as defined above.

The process may be effected in conventional manner for analogous dihydropyridine syntheses, e.g. according to Hantzsch. When the moiety —HC=Y is formyl and when it is desired to produce a compound of formula I, wherein $R_2$ is identical to $R_5$ and $R_3$ is identical to $R_4$, it is convenient to react a compound of formula II with a compound of formula IV,

$$R_5CO—CH_2—CO—R_4 \qquad IV$$

wherein $R_4$ and $R_5$ are as defined above, in the presence of a compound of formula V,

$$H_2NR_1 \qquad V$$

wherein $R_1$ is as defined above.

Preferably at least 2 moles of a compound of formula IV per mole of a compound of formula II are present. Alternatively a compound of formula II may be reacted with a compound of formula VI,

$$R_5—C(NH—R_1)=CH—CO—R_4 \qquad VI$$

wherein $R_1$, $R_4$ and $R_5$ are as defined above.

Preferably at least 2 moles of a compound of formula VI per mole of a compound of formula II are present. Preferably also $R_1$ is hydrogen.

When the moiety —HC=Y is formyl and preferably when it is desired to produce a compound of formula I wherein $R_2$ is different to $R_5$ and/or $R_3$ is different to $R_4$, it is also possible to react such a compound of formula II with a compound of formula IV and a compound of formula VII,

$$R_2—C(NH—R_1)=CH—CO—R_3 \qquad VII$$

wherein $R_2$, $R_1$ and $R_3$ are as defined above.

It will be appreciated that a compound of formula VI may be formed as an intermediate during the reaction of a compound of formula IV and a compound of formula V. A compound of formula II, wherein —HC=Y is a radical ii) or iii), may be formed as an intermediate in the above reactions. They may however be produced by different processes.

Alternatively or particularly for the production of a compound of formula I, wherein $R_2$ is different to $R_5$ and/or $R_3$ is different to $R_4$, it is convenient to react a compound of formula II, wherein the moiety —HC=Y is a radical ii) with a compound of formula IV or VI, and where appropriate, with a compound of formula V. A compound of formula II, wherein the moiety —HC=Y is a radical iii) may be an intermediate.

In the above reactions it is possible in certain instances when $R_2$, $R_3$, $R_4$ and $R_5$ are not identical that more than one isomer of formula I may be formed. If so these may be separated in conventional manner, e.g. by thin layer chromatography.

When the starting material is a compound of formula II, wherein —HC=Y is a radical iii), the

reaction is a ring cyclisation. When Z and Z′ are both oxygen, then an amine of formula V should be present.

However, all the above reactions may be effected under the same conditions.

The reaction may be effected conveniently in solution. A suitable solvent is water, ethanol, dioxane, dimethyl formamide, dimethyl sulphoxide, pyridine or glacial acetic acid. Suitable reaction temperatures may be from 20 to 160°C, preferably from 60 to 120°C.

Insofar as the production of starting materials is not particularly described these compounds are known or may be produced in analogous manner to known compounds.

In the following Examples the temperatures given are in degrees Centigrade and are uncorrected.

Example 1

4-(2,1,3-Benzoxadiazol-4-yl)-2,6-dimethyl-1,4-dihydro-pyridine-3,5-dicarboxylic acid diethyl ester

3.2 g of 2,1,3-benzoxadiazole-4-aldehyde, 5.7 g of acetoacetic acid ethyl ester, 2.5 ml of concentrated ammonia and 10 ml of ethanol are refluxed for 6 hours. The mixture is subsequently evaporated and the residual oil is chromatographed on silica gel with chloroform/acetic acid ester (9:1) to yield the title compound. The product is recrystallised from toluene, m.p. 153—155°.

By using the process described in Example 1, and corresponding starting compounds, e.g. a compound of formula II, wherein —HC=Y is a radical i) and compounds of formula IV and V, and for Examples 18 and 19 a compound of formula II, wherein —HC=Y is a radical ii), wherein Z is oxygen and a compound of formula VI, the following compounds of formula I may be obtained, wherein y indicates the position of the dihydropyridine moiety:

| Example | $R_1$ | $R_2$ | $R_3$ | $R_4$ | $R_5$ | $R_6$ | X | y | m.p. |
|---|---|---|---|---|---|---|---|---|---|
| 2 | H | $CH_3$ | $OC_2H_5$ | $OC_2H_5$ | $CH_3$ | H | S | 4 | 146—148 |
| 3 | H | $CH_3$ | $OC(CH_3)_3$ | $OC(CH_3)_3$ | $CH_3$ | H | S | 4 | 193—199 |
| 4 | H | $CH_3$ | $OCH_3$ | $OCH_3$ | $CH_3$ | 7—Cl | O | 4 | 207—211 |
| 5 | H | $CH_3$ | $OCH_3$ | $OCH_3$ | $CH_3$ | H | S | 4 | 215—216 |
| 6 | H | $CH_3$ | $OC_2H_5$ | $OC_2H_5$ | $CH_3$ | 5—$OCH_3$ | S | 4 | 201—203 |
| 7 | H | $CH_3$ | $OC_2H_5$ | $OC_2H_5$ | $CH_3$ | 7—Cl | S | 4 | 135—155 |
| 8 | H | $CH_3$ | $OC_2H_5$ | $OC_2H_5$ | $CH_3$ | H | S | 5 | 152—153 |
| 9 | H | $CH_3$ | $OC_2H_5$ | $OC_2H_5$ | $CH_3$ | 4—Cl | S | 5 | 198—200 |
| 10 | H | $CH_3$ | $C(CH_3)_3$ | $C(CH_3)_3$ | $CH_3$ | H | S | 4 | |
| 11 | H | $CH_3$ | $CH_3$ | $CH_3$ | $CH_3$ | 7—Cl | O | 4 | |
| 12 | H | $CH_3$ | $CH_3$ | $CH_3$ | $CH_3$ | H | S | 4 | |
| 13 | H | $CH_3$ | $C_2H_5$ | $C_2H_5$ | $CH_3$ | 5—$OCH_3$ | S | 4 | |
| 14 | H | $CH_3$ | $C_2H_5$ | $C_2H_5$ | $CH_3$ | 7—Cl | S | 4 | |
| 15 | H | $CH_3$ | $C_2H_5$ | $C_2H_5$ | $CH_3$ | H | S | 5 | |
| 16 | H | $CH_3$ | $C_2H_5$ | $C_2H_5$ | $CH_3$ | 4—Cl | S | 5 | |
| 17 | H | $CH_3$ | $CH_3$ | $CH_3$ | $CH_3$ | H | O | 4 | |
| 18 | H | $CH_3$ | $CH_3$ | $OC_2H_5$ | $CH_3$ | H | O | 4 | |
| 19 | H | $CH_3$ | $CH_3$ | $OC_2H_5$ | $CH_3$ | H | S | 4 | |

The compounds of formula I exhibit pharmacological activity. In particular, they lead to a dilation of the coronary vessels as demonstrated by the results of tests measuring the blood flow to the myocardium of an anaesthetised cat by means of the microsphere method upon the administration of the active substance i.v. or i.d. The compounds of formula I also possess a favourable effect against

angina pectoris, as shown by the increase of the coronary flow of an anaesthetised cat upon administration of the active substance.

The compounds of formula I are therefore indicated for use in the treatment of coronary insufficiency. For this use an indicated daily dose is from about 5 to 100 mg, conveniently administered in divided doses 2 to 4 times a day in unit dosage form containing from about 1.25 to about 50 mg, or in sustained release form.

Additionally, the compounds of formula I exhibit antihypertensive activity, as indicated in standard tests, e.g. in the Grollman rat test [see A. Grollman, Proc. Soc. Expt. Biol. and Med. *57*, 104 (1944)] on s.c. administration of from 0.1 to 10 mg/kg animal body weight of the compounds.

The compounds are therefore further indicated for use as anti-hypertensive agents. For this use an indicated daily dose is from about 5 to about 1000 mg, conveniently given in divided doses 2 to 4 times a day in unit dosage form containing about 1.25 mg to about 500 mg, or in sustained release form.

The compounds of formula I may be administered in the form of a pharmaceutical composition. The present invention accordingly provides a pharmaceutical composition comprising a compound of formula I in association with a pharmaceutical carrier or diluent. Such compositions may be prepared by conventional techniques to be in conventional forms, for example capsules or tablets.

The compounds of Examples 1 and 2 are the preferred compounds. The coronary insufficiency utility is preferred utility.

In a group of compounds $R_1$ is hydrogen, alkyl, alkenyl, cycloalkyl of 3 to 6 carbon atoms or phenylalkyl; the phenyl ring being unsubstituted or substituted by one, two or three substituents chosen from one or two halogen radicals, one or two alkyl groups of 1 to 4 carbon atoms, one to three alkoxy groups of 1 to 4 carbon atoms; $R_3$ and $R_4$, indpendently, are alkyl, alkenyl, cycloalkyl of 3 to 6 carbon atoms, alkoxy, hydroxyalkoxy of 2 to 6 carbon atoms, alkoxyalkoxy of 3 to 6 carbon atoms, hydroxyalkoxyalkoxy of 4 to 8 carbon atoms, alkenyloxy, or cycloalkyloxy of 3 to 6 carbon atoms, and $R_6$ is other than alkylsulfonyl.

Conveniently $R_1$ is hydrogen, $R_2$ and $R_5$ are each alkyl, especially methyl, $R_3$ and $R_4$ are each alkoxy, especially ethoxy, $R_6$ is hydrogen or halogen, especially chlorine, especially in the 4 position, the dihydropyridine moiety is in the 4 or 5 position, and X is S.

Alternatively conveniently $R_1$ is hydrogen, $R_2$ and $R_5$ are each alkyl, especially methyl, $R_3$ and $R_4$ are each alkyl or alkoxy, especially methyl, ethyl, tert. butyl, methoxy, ethoxy or tert. butyloxy, R— is hydrogen or halogen, especially chlorine, or alkoxy, especially methoxy, the dihydropyridine moiety is in the 4 or 5 position and R— is in the 4, 5 or 7 position.

## Claims

1. A process for the production of a compound of formula I,

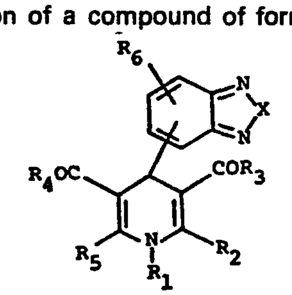

wherein

$R_1$ is hydrogen, alkyl of 1 to 6 carbon atoms, alkenyl or alkynyl of 3 to 6 carbon atoms, cycloalkyl of 3 to 7 carbon atoms, cycloalkylalkyl of 4 to 8 carbon atoms, phenylalkyl of 7 to 9 carbon atoms or phenylalkenyl of 9 to 12 carbon atoms, the phenyl ring being unsubstituted or mono, di- or trisubstituted independently by halogen, hydroxy or alkyl or alkoxy or 1 to 4 carbon atoms,

$R_2$ and $R_5$, independently, are hydrogen or alkyl of 1 to 6 carbon atoms,

$R_3$ and $R_4$ independently, are alkyl of 1 to 6 carbon atoms, alkenyl or alkynyl of 3 to 6 carbon atoms, cycloalkyl of 3 to 7 carbon atoms, cycloalkylalkyl of 4 to 8 carbon atoms, alkoxy of 1 to 6 carbon atoms, hydroxyalkoxy of 2 to 6 carbon atoms, alkoxyalkoxy of 3 to 6 carbon atoms, hydroxyalkoxyalkoxy of 4 to 8 carbon atoms, alkenyloxy or alkynyloxy of 3 to 6 carbon atoms, cycloalkyloxy of 3 to 7 carbon atoms or cycloalkylalkoxy of 4 to 8 carbon atoms,

$R^6$ is hydrogen, halogen, alkyl or alkoxy or alkylthio or alkylsulfonyl, each of 1 to 4 carbon atoms, trifluoromethyl, nitro or hydroxy, and

X is oxygen or sulphur,

which comprises chemically converting the moiety —HC=Y in a compound of formula II,

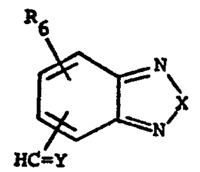

II

wherein $R_6$ and X are as defined above, and —HC=Y is
i) formyl,
ii) a radical of formula

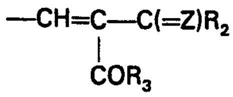

or
iii) a radical of formula

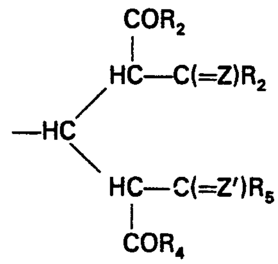

wherein z and z' are independently oxygen or $NR_1$, and $R_1$ to $R_5$ are as defined above, into a moiety of formula III,

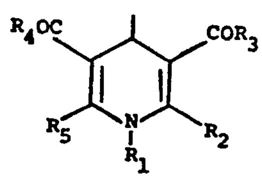

III

wherein $R_1$ to $R_5$ are as defined above.

2. A compound of formula I, as defined in claim 1.

3. A compound of claim 2, wherein $R_1$ is hydrogen.

4. A compound of claim 2, wherein $R_2$ and $R_5$ are methyl.

5. A compound of claim 2, wherein at least one of $R_3$ and $R_4$ is alkoxy or alkoxyalkoxy.

6. A compound of claim 2, wherein $R_6$ is hydrogen.

7. A compound of claim 2, wherein $R_1$ is hydrogen, alkyl, alkenyl, cycloalkyl of 3 to 6 carbon atoms or phenylalkyl, the phenyl ring being unsubstituted or substituted by one, two or three substituents chosen from one or two halogen radicals, one or two alkyl groups of 1 to 4 carbon atoms, and one to three alkoxy groups of 1 to 4 carbon atoms, $R_3$ and $R_4$, independently, are alkyl, alkenyl, cycloalkyl of 3 to 6 carbon atoms, alkoxy, hydroxyalkoxy of 2 to 6 carbon atoms, alkoxyalkoxy of 3 to 6 carbon atoms, hydroxyalkoxyalkoxy of 4 to 8 carbon atoms, alkenyloxy or cycloalkoxy of 3 to 6 carbon atoms, and $R_6$ is other than alkylsulphonyl.

8. A compound of claim 7, wherein $R_1$ is hydrogen, $R_2$ and $R_5$ are each alkyl, $R_3$ and $R_4$ are each alkyl or alkoxy, $R_6$ is hydrogen or halogen or alkoxy, the dihydropyridine moiety is in the 4 or 5 position and $R_6$ is in the 4, 5 or 7 position.

9. 4-(2,1,3-benzoxadiazol-4-yl)-2,6-dimethyl-1,4-dihydro-pyridine-3,5-dicarboxylic acid diethyl ester.

10. A pharmaceutical composition comprising a compound of anyone of claims 2 to 9 in association with a pharmaceutical carrier or diluent.

11. A compound of formula I as defined in anyone of claims 2 to 9 for use as a pharmaceutical.

**Revendications**

1. Un Procédé de préparation d'un composé de formule I

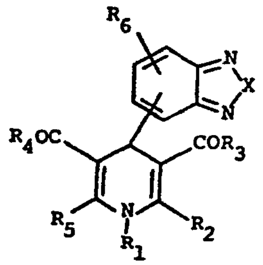

(I)

dans laquelle

$R_1$ représente l'hydrogène, un groupe alkyle contenant de 1 à 6 atomes de carbone, alcényle ou alcynyle contenant de 3 à 6 atomes de carbone, cycloalkyle contenant de 3 à 7 atomes de carbone, cycloalkylalkyle contenant de 4 à 8 atomes de carbone, phénylalkyle contenant de 7 à 9 atomes de carbone ou phénylalcényle contenant de 9 à 12 atomes de carbone, le cycle phénylique étant non substitué ou mono-, di- ou trisubstitué indépendamment par de l'halogène, un groupe hydroxy ou un groupe alkyle ou alcoxy contenant de 1 à 4 atomes de carbone,

$R_2$ et $R_5$ signifient, indépendamment l'un de l'autre, l'hydrogène ou un groupe alkyle contenant de 1 à 6 atomes de carbone,

$R_3$ et $R_2$ signifient, indépendamment l'un de l'autre, un groupe alkyle contenant de 1 à 6 atomes de carbone, alcényle ou alcynyle contenant de 3 à 6 atomes de carbone, cycloalkyle contenant de 3 à 7 atomes de carbone, cycloalkylalkyle contenant de 4 à 8 atomes de carbone, alcoxy contenant de 1 à 6 atomes de carbone, hydroxyalcoxy contenant de 2 à 6 atomes de carbone, alcoxyalcoxy contenant de 3 à 6 atomes de carbone, hydroxyalcoxyalcoxy contenant de 4 à 8 atomes de carbone, alcényloxy ou alcynyloxy contenant de 3 à 6 atomes de carbone, cycloalkyloxy contenant de 3 à 7 atomes de carbone ou cycloalkylalcoxy contenant de 4 à 8 atomes de carbone,

$R_6$ signifie l'hydrogène, un halogène, un groupe alkyle ou alcoxy ou alkylthio ou alkylsulfonyle contenant chacun de 1 à 4 atomes de carbone, trifluorométhyle, nitro ou hydroxy, et

X signifie l'oxygène ou le soufre,

lequel comprend la conversion chimique du reste —HC=Y présent dans un composé de formule II

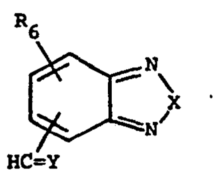

(II)

dand laquelle $R_6$ et X ont les significations donns ci-dessus, et
—HC=Y signifie
i) un groupe formyle,
ii) un radical de formule

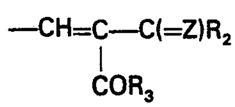

ou
iii) un radical de formule

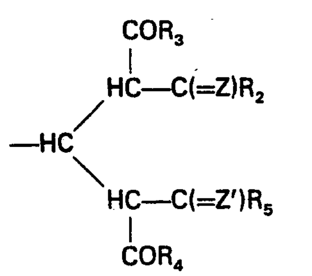

où et Z' représentent, indépendamment l'un de l'autre, un atome d'oxygène ou un reste NR, et $R_1$ à $R_5$ ont les significations données ci-dessus, en un reste de formule III

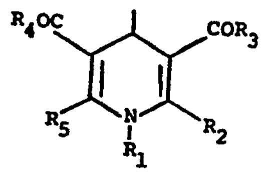

(III)

dans laquelle $R_1$ à $R_5$ ont les significations données-ci-dessus.

2. Un composé de formule I, tel que défini à la revendication 1.

3. Un composé de la revendication 2, dans lequel $R_1$ signifie l'hydrogène.

4. Un composé de la revendication 2, dans lequel $R_2$ et $R_5$ signifient un groupe méthyle.

5. Un composé de la revendication 2, dans lequel l'un moins des symboles $R_4$ et $R_4$ signifie un groupe alcoxy ou alcoxyalcoxy.

6. Un composé de la revendication 2, dans lequel $R_6$ signifie l'hydrogène.

7. Un composé de la revendication 2, dans lequel $R_1$ signifie l'hydrogène, un groupe alkyle, alcényle, cycloalkyle contenant de 3 à 6 atomes de carbone ou phénylalkyle, le reste phénylique étant non substitué ou substitué par un, deux ou trois substituants choisis parmi 1 ou 2 radicaux halogène, un ou deux groupes alkyle contenant de 1 à 4 atomes de carbone, et un à trois groupes alcoxy contenant de 1 à 4 atomes de carbone, $R_3$ et $R_4$ signifient, indépendamment l'un de l'autre, un groupe alkyle, alcényle, cycloalkyle contenant de 3 à 6 atomes de carbone, alcoxy, hydroxyalcoxy contenant de 2 à 6 atomes de carbone, alcoxyalcoxy contenant de 3 à 6 atomes de carbone, hydroxyalcoxyalcoxy contenant de 4 à 8 atomes de carbone, alcényloxy ou cycloalkyloxy contenant de 3 à 6 atomes de carbone, et $R_6$ a une signification autre que alkylsulfonyle.

8. Un composé de la revendication 7, dans lequel $R_1$ signifie l'hydrogène, $R_2$ et $R_5$ signifient chacun un groupe alkyle, $R_3$ et $R_4$ représentent chacun un groupe alkyle ou alcoxy, $R_6$ représente l'hydrogène ou un halogène ou un groupe alcoxy, le reste dihydropyridine est en position 4 ou 5 et $R_6$ est en position 4, 5 ou 7.

9. L'ester diéthylique de l'acide 4-(2,1,3-benzoxadiazole-4-yl)-2,6-diméthyl-1,4-dihydropyridine-3,5-dicarboxylique.

10. Une composition pharmaceutique comprenant un composé de l'une quelconque des revendications 2 à 9 en association avec un excipient ou un diluant pharmaceutique.

11. Un composé de formule I tel que défini à l'une quelconque des revendications 2 à 9, pour l'utilisation comme médicament.

**Patentansprüche**

1. Verfahren zur Herstellung einer Verbindung der Formel I,

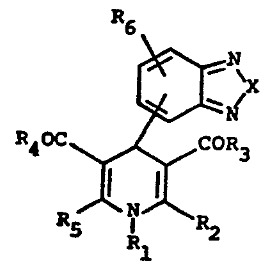

I

worin

$R_1$ für Wasserstoff, Alkyl mit 1—6 Kohlenstoffatomen, Alkenyl oder Alkinyl mit jeweils 3—6 Kohlenstoffatomen, Cycloalkyl mit 3—7 Kohlenstoffatomen, Cycloalkylalkyl mit 4—8 Kohlenstoffatomen, Phenylalkyl mit 7—9 Kohlenstoffatomen oder Phenylalkenyl mit 9—12 Kohlenstoffatomen, wobei der Phenylring unsubstituiert oder durch ein, zwei oder drei Substituenten, die unabhängig voneinander Halogen, Hydroxy, Alkyl oder Alkoxy mit jeweils 1—4 Kohlenstoffatomen bedeuten, substituiert ist, steht,

$R_2$ und $R_5$ unabhängig voneinander Wasserstoff oder Alkyl mit 1—6 Kohlenstoffatomen bedeuten,

$R_3$ und $R_4$ unabhängig voneinander für Alkyl mit 1—6 Kohlenstoffatomen, Alkenyl oder Alkinyl mit jeweils 3—6 Kohlenstoffatomen, Cycloalkyl mit 3—7 Kohlenstoffatomen, Cycloalkylalkyl mit 4—8 Kohlenstoffatomen, Alkoxy mit 1—6 Kohlenstoffatomen, Hydroxyalkoxy mit 2—6 Kohlenstoffatomen, Alkoxyalkoxy mit 3—6 Kohlenstoffatomen, Hydroxyalkoxyalkoxy mit 4—8 Kohlenstoffatomen, Alkenyloxy oder Alkinyloxy mit jeweils 3—6 Kohlenstoffatomen, Cycloalkyloxy mit 3—7 Kohlenstoffatomen oder Cycloalkylalkoxy mit 4—8 Kohlenstoffatomen, stehen,

$R_6$ Wasserstoff, Halogen, Alkyl, Alkoxy, Alkythio oder Alkylsulfonyl mit jeweils 1—4 Kohlenstoffatomen, Trifluormethyl, Nitro oder Hydroxy, und

X Sauerstoff oder Schwefel bedeuten, dadurch gekennzeichnet, dass man in einer Verbindung der Formel II,

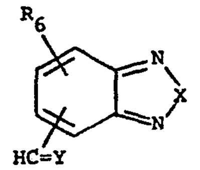

II

worin $R_6$ und X obige Bedeutung besitzen, und —HC=Y
i) Formyl,
ii) eine Gruppe der Formel

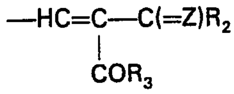

oder
iii) eine Gruppe der Formel

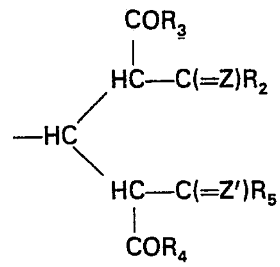

bedeutet, worin Z und Z' unabhängig voneinander für Sauerstoff oder $NR_1$ stehen und $R_1$—$R_5$ obige Bedeutung besitzen,
die Gruppe —HC=Y in eine Gruppe der Formel III,

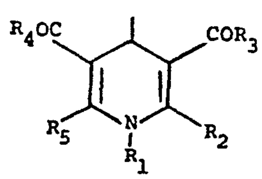

III

worin $R_1$—$R_5$ obige Bedeutung besitzen, chemisch überführt.

2. Eine Verbindung der Formel I, wie im Anspruch 1 definiert.

3. Eine Verbindung nach Anspruch 2, worin $R_1$ Wasserstoff bedeutet.

4. Eine Verbindung nach Anspruch 2, worin $R_2$ und $R_5$ Methyl bedeuten.

5. Eine Verbindung nach Anspruch 2, worin mindestens einer der Substituenten $R_3$ und $R_4$ Alkoxy oder Alkoxyalkoxy bedeutet.

6. Eine Verbindung nach Anspruch 2, worin $R_6$ Wasserstoff bedeutet.

7. Eine Verbindung nach Anspruch 2, worin $R_1$ für Wasserstoff, Alkyl, Alkenyl, Cycloalkyl mit 3—6 Kohlenstoffatomen oder Phenylalkyl steht, wobei der Phenylring unsubstituiert oder durch ein, zwei oder drei Substituenten, ausgewählt von ein oder zwei Halogenatomen, ein oder zwei Alkylgruppen mit 1—4 Kohlenstoffatomen und ein bis drei Alkoxygruppen mit 1—4 Kohlenstoffatomen substituiert ist, $R_3$ und $R_4$ unabhängig voneinander für Alkyl, Alkenyl, Cycloalkyl mit 3—6 Kohlenstoffatomen, Alkoxy, Hydroxyalkoxy mit 2—6 Kohlenstoffatomen, Alkoxyalkoxy mit 3—6 Kohlenstoffatomen, Hydroxyalkoxyalkoxy mit 4—8 Kohlenstoffatomen, Alkenyloxy oder Cycloalkyloxy mit 3—6 Kohlenstoffatomen stehen, und $R_6$ nicht Alkylsulfonyl bedeutet.

8. Eine Verbindung nach Anspruch 7, worin $R_1$ für Wasserstoff, $R_2$ und $R_5$ jeweils für Alkyl, $R_3$ und $R_4$ jeweils für Alkyl oder Alkoxy, $R_6$ für Wasserstoff oder Halogen oder Alkoxy stehen, der Dihydropyridinrest in 4- oder 5-Stellung, und $R_6$ in 4-, 5- oder 7-Stellung stehen.

9. 4-(2,1,3-Benzoxadiazol-4-yl)-2,6-dimethyl-1,4-dihydropyridin-3,5-dicarbonsäurediäthylester.

10. Pharmazeutische Zusammensetzung enthaltend eine Verbindung nach einem der Ansprüche 2 bis 9 zusammen mit pharmazeutischen Träger- und Verdünnungsmitteln.

11. Eine Verbindung der Formel 1 nach einem der Ansprüche 2 bis 9 zur Verwendung als Pharmazeutikum.